# EUROPÄISCHE PATENTSCHRIFT

(11) **EP 0 590 452 B1**
(45) Veröffentlichungstag und Bekanntmachung des Hinweises auf die Patenterteilung: **15.01.1997**
(21) Anmeldenummer: 93115063.5
(22) Anmeldetag: 20.09.1993
(51) Int. Cl.: A61F 5/41

(54) **Aussenprothese zur Stützung des Penis**
External prosthesis for supporting a penis
Prothèse externe pour soutenir le pénis

(30) Priorität: 26.09.1992 DE 9212995 U
(43) Veröffentlichungstag der Anmeldung: 06.04.1994
(73) Patentinhaber: Sickinger, Horst, Dipl.-Ing. Prof., D-61231 Bad Nauheim (DE)
(72) Erfinder: Sickinger, Horst, Dipl.-Ing. Prof., D-61231 Bad Nauheim (DE)
(74) Vertreter: Schlagwein, Udo, Dipl.-Ing.

(56) Entgegenhaltungen:
- AT-B- 66 594
- CH-A- 60 397
- DE-C- 596 763
- US-A- 4 653 484

## Beschreibung

Die Erfindung betrifft eine Außenprothese zur Stützung des Penis bei teilweiser oder völliger Impotenz, bestehend aus einem den Penis umfassenden, steif ausgebildeten, in der Vagina gleitfähigen, als Korb ausgebildeten Hohlkörper, der auf seiner bei Benutzung dem Körper zugewandten Seite zum Durchführen des Skrotums von oben her einen in Horizontalebene offenen Bügel hat und nahe seines vorderen Endes eine Auflage für den Penis aufweist.

Eine Außenprothese der vorstehenden Art ist Gegenstand der DE-B-405 400. Die bekannte Außenprothese besteht aus einem korbartigem Gebilde aus Zelluloid, dessen Kanten abgerundet sind. An dem dem Benutzer zugewandten Ende ist an dem Korb ein Bügel in Form einer Gummischlaufe vorgesehen, der quer zur Haupterstreckungsrichtung der Außenprothese offen ist und durch den hindurch zum Anlegen der Außenprothese das Skrotum geführt wird.

Nachteilig bei der bekannten Prothese ist es, daß sie lediglich eine stützende Wirkung hat, jedoch weder die Erektionsfähigkeit des Penis zu fördern noch eine erektive Füllung und Spannung herbeizuführen vermag. Deshalb ist die Verwendung einer solchen Prothese kaum geeignet, einen Orgasmus und damit Ejakulation zu erreichen.

Durch die AT-C-1 12 737 ist auch schon eine Außenprothese bekannt geworden, welche zusätzlich zu einer stützenden Wirkung eine die erektive Füllung fördernde Wirkung aufweisen soll. Hierzu tragen zwei seitlich entlang des Penis zu führende, steife Schienen jeweils ein an den Rand der Eichel anzulegendes Polster. Diese Polster sind durch ein über die Eichel zu spannendes Gummiband miteinander verbunden. Das Gummiband vermag dort die Eichel mit Vorspannkraft teilweise zu umschließen und dadurch in Folge eines Einschnüreffektes einen Blutstau hervorzurufen, was zu einer größeren Blutzufuhr in den Schwellkörpern und damit erektiv wirkt. Zum Halten der Außenprothese hat diese an ihrem rückwärtigen Ende einen nach oben hin offenen Bügel, über den nach dem Einlegen des Penis ebenfalls ein Gummiband zu spannen ist.

Nachteilig bei dieser Außenprothese ist es, daß sie sich nicht ausreichend fest am Penis befestigen läßt, so daß sie nur mit erhöhter Aufmerksamkeit zu benutzen ist. Weiterhin hat sie nur eine sehr beschränkte, stützende Wirkung, da sie nicht als ein korbartiges Gebilde gestaltet ist. Die verbundenen, einzelnen Schienen stellen für die Frau eine große Verletzungsgefahr dar. Die Berührung der Polster mit der Eichel kann beim Koitus schmerzhaft stören.

Durch die CH-A-0 060 397 ist es auch schon bekannt, an der vorderen Seite einer Außenprothese eine gummielastische Schlaufe anzuordnen, welche bei Gebrauch der Außenprothese zur Fixierung der Eichel auf den Penis über die Eichel geschoben wird.

Der Erfindung liegt das Problem zugrunde, eine Außenprothese der eingangs genannten Art so auszubilden, daß sie zusätzlich zu ihrer stützenden Wirkung die erektive Füllung zu fördern vermag und dadurch zugleich eine optimale Verbindung des Penis mit der Außenprothese herstellt.

Dieses Problem wird erfindungsgemäß dadurch gelöst, daß die Auflage mit einer von oben her über den Hals der Eichel des Penis zu ziehende, diesen auf der Auflage fixierende und einschnürende Schlaufe versehen und die Schlaufe Teil einer geschlossenen Rundgummischnur ist, welche zum Spannen der Schlaufe in eine Aufhängung des rückwärtigen Ringes einhängbar ist.

Da die erfindungsgemäße Außenprothese auf ihrer bei Benutzung dem Körper zugewandten Seite zum Durchführen des Skrotums von oben her einen in Horizontalebene offenen Bügel hat, findet sie ohne sonstige Haltemittel am Penis guten Halt. Sie ist deshalb angenehm zu tragen und kann ohne Schwierigkeiten angelegt werden.

Die im vorderen Bereich von oben her über den Hals der Eichel des Penis zu ziehende, diesen auf der Auflage fixierende und einschnürende Schlaufe hat eine Doppelfunktion. Sie fixiert den Penis auf der vorderen Auflage und sie bewirkt gleichzeitig einen Einschnüreffekt auf den vorderen Teil des Penis. Dieser fördert einen Blutstau, was das Entstehen einer Erektion begünstigt. Dank der erfindungsgemäßen Außenprothese kann somit die erektionsartige Füllung wieder herbeigeführt werden, was gesundheitlich vorteilhaft ist, da die bei älteren Männern häufig auftretenden Prostataerkrankungen (Erweiterung) möglicherweise auch durch Abnahme der Ejakulationshäufigkeit bedingt sind.

Da gemäß der Erfindung die Schlaufe Teil einer geschlossenen Rundgummischnur ist, welche zum Spannen der Schlaufe in eine Aufhängung des rückwärtigen Ringes einhängbar ist, kann die Außenprothese konstruktiv besonders einfach aufgebaut sein. Zugleich ist ihr Spanneffekt sehr einfach herbeizuführen.

Zur weiteren Verbesserung des Haltes der Außenprothese trägt es bei, wenn der Bügel fest und unbeweglich ausgebildet ist und ihm sich zum Durchführen des Penis ein in Vertikalebene offener Ring anschließt.

Die erektive Spannung wird im besonders starken Maße gefördert, wenn die Aufhängungen an zwei Seiten des Ringes vorgesehen und zur Erzeugung eines zweiten, den Penis an seiner Basis durch die Rundgummischnur einschnürenden Bereiches ausgebildet sind.

Ein Einklemmen von Hautbereichen des Frenulum praeputii (Bändchen) durch die vordere Schlaufe kann auf einfache Weise dadurch verhindert werden, daß die Auflage zur Durchführung der Schlaufe ein in Querrichtung verlaufendes Langloch hat. Ein solches Langloch ermöglicht zudem die Einführung einer geschlossenen Rundgummischnur und deshalb einen leichten Austausch der Rundgummischnur.

Ausreichend hohe Einschnürungskräfte lassen sich ohne unangenehmes Druckgefühl erzielen, wenn die Schlaufe aus querelastischer Moosgummischnur besteht. Eine solche Schlaufe blockiert nicht den Durchfluß von Urin und Sperma.

Die mit der Schlaufe zu erzielenden Spannkräfte können auf ein konstruktiv festgelegtes Maß begrenzt werden, indem der Querschnitt der Schlaufe größer ist als der des Langloches und der Schnur, wodurch ein Anschlag entsteht.

Ein Einziehen der Schlaufe in das Langloch kann auf einfache Weise dadurch vermieden werden, daß auf der Rundgummischnur zu beiden Seiten der Schlaufe jeweils eine gegen den jeweiligen Beginn der Schlaufe anliegende Vollgummischeibe sitzt.

Von Vorteil ist es auch, wenn der Korb zusätzlich zu dem Ring eine den Penis auf seiner Oberseite übergreifende Brücke in einem mittleren Bereich hat. Eine solche Brücke wirkt zusätzlich stabilisierend für die Korbsteifigkeit und fördert durch das entstehende Staboval die Gleitung in der Vagina.

Weitere für die Erfindung wesentliche Merkmale der Außenprothese sind in den Ansprüchen 9, 10 und 11 gekennzeichnet. Ihre besonderen Vorteile ergeben sich aus der nachfolgenden Beschreibung.

Die Erfindung läßt zahlreiche Ausführungsformen zu. Zur weiteren Verdeutlichung ihres Grundprinzips ist eine davon in der Zeichnung dargestellt und wird nachfolgend beschrieben. Diese zeigt in
- Fig.1: eine Seitenansicht der Außenprothese im benutzten Zustand,
- Fig.2: eine Draufsicht auf die Außenprothese,
- Fig.3: eine Ansicht von unten auf die Außenprothese.
- Fig.4: eine im Maßstab vergrößerte Darstellung eines Endes einer Schlaufe der Vorrichtung.

Die erfindungsgemäße Außenprothese besteht aus einem Korb 1, in welchem der Penis 2 gehalten ist. Dieser Korb 1 hat an seinem rückwärtigen Ende einen in der Horizontalebene offenen Bügel 3, durch den bei Gebrauch von oben her das Skrotum 4 durchzuführen ist, nachdem zuvor der Penis 2 durch einen in der Vertikalebene offenen Ring 5 geführt wurde. Der Penis 2 stützt sich am Schluß im vorderen Bereich von oben her auf einer Auflage 6 ab, die er nach vorn hin und seitlich mit seiner Eichel 7 überragt. Durch diese Gestaltung bildet der Korb 1 ohne zusätzliche Hilfsmittel mit dem Penis 2 eine erektionsartige Einheit.

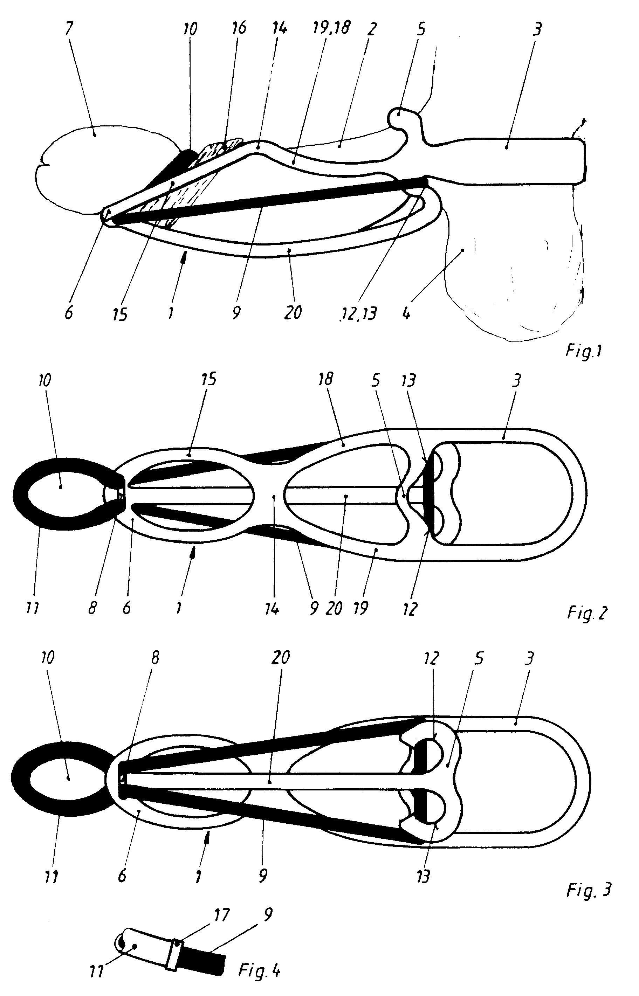

In der Auflage 6 ist ein in den Figuren 2 und 3 zu sehendes, in Querrichtung verlaufendes Langloch 8 vorgesehen, durch welches eine geschlossene Rundgummischnur 9 führt. Diese bildet oberhalb der Auflage 6 eine Schlaufe 10, die aus Moosgummischnur 11 besteht. Diese ist im Querschnitt so bemessen, daß sie im Gegensatz zur Rundgummischnur 9 nicht durch das Langloch 8 paßt.

Die Figur 2 läßt besonders deutlich erkennen, daß vom Ring 5 drei Längsstäbe 18, 19, 20 zu einem vorderen Oval 15 führen, welches, was Figur 1 zeigt, schräg nach vorn unten geneigt verläuft. Die zwei Längsstäbe 18, 19 sind im mittleren Bereich durch die Brücke 14 miteinander verbunden.

Wie die Figur 4 zeigt, sind an den Enden der Moosgummischnur 11 sind Vollgummischeiben 17 mit der Dicke der Moosgummischnur 11 eingefügt, um zu verhindern, daß die Moosgummischnur 11 in das Langloch 8 eingezogen werden kann (Anschlag).

Bei Gebrauch wird die Schlaufe 10 über den Hals der Eichel 7 geführt, was in Figur 1 zu sehen ist. Dann wird die Rundgummischnur 9 im rückwärtigen Teil des Korbes 1 in die Aufhängungen 12, 13 eingehangen und dadurch die Schlaufe 10 gespannt. Hierdurch tritt ein Strangulationseffekt ein, der zu einem Blutstau führt und dadurch eine Erektion fördert, sowohl vorn an der Eichel 7, als auch hinten an der Basis des Penis 2.

Die Erfindung ahmt die anatomischen Gegebenheiten des männlichen Organs insofern nach, daß die drei nicht mehr funktionierenden inneren Schwellkörper außen durch die drei Stäbe 18, 19, 20 an gleicher Stelle (zwei oben, einer unten) ersetzt werden, die vorne am Fixierungspunkt der Eichel 7 zusammenlaufen und mit der dabei entstehenden stumpfen Pyramidenspitze beim Eindringen und bei der Bewegung in der Vagina keine Gefahr für die Frau bilden.

Die erektionsartige Steife und Stellung wird dadurch erreicht, daß, wie bei einer echten Erektion, der Penis 2 gegen die Stäbe 18, 19, 20 gespannt wird, was durch die Fixierung der Eichel 7 an der einzig möglichen Stelle, nämlich am Ansatz des Frenulum praeputii nach vorheriger Spannung der Rundgummischnur 9 erreicht wird. Durch die kombinierte Anordnung von Skrotum-Bügel mit dem Basisring für die drei Längsstäbe 18, 19, 20 wird die natürliche Befestigung der drei Schwellkörper am Schambein nachgeahmt und somit Steifigkeit und Stellung einer wirklichen Erektion erreicht.

Zur Erhöhung der Sicherheit gegen Schmerz und Verletzung beim Eindringen und Gleiten in der Vagina sind die beiden oberen Längsstäbe 18, 19 etwa in Längenmitte durch die Brücke 14 verbunden, die zusammen mit dem vorderen Teil der oberen Längsstäbe 18, 19 das Oval 15 bildet, durch das die Eichel 7 durchgeführt wird. Dieses liegende Oval 15 bietet von der Form her hohe Gleitsicherheit und die Fähigkeit zum Öffnen der Scheide, so daß das Gerät auch ohne Penis 2 in die Scheide eingeführt werden könnte. Dies ist von sehr großer Bedeutung, da der impotente Penis 2 mindestens zunächst wenig materielle Substanz aufweist. Durch eine bananenförmige Biegung des Korbes 1 nach vorne oben wird die Brücke 14 niedrig gehalten und somit unangenehme Empfindungen in der Scheide vermieden. Das Oval 15 ist etwa zweimal länger als für die Aufnahme der Eichel 7 nötig wäre, damit eine Berührung des Eichelrandes mit dem Oval 15 während des Gebrauchs und damit schmerzhafte Klemmungen ausgeschlossen sind. Desgleichen ergibt sich so genügend Platz, um die zurückgestreifte Vorhaut 16 klemmungsfrei anzuordnen.

Der Ring 5 dient nicht, wie bei anderen Erfindung, direkt der strangulierenden Einschnürung, sondern vornehmlich der Aufnahme der drei Längsstäbe 18, 19, 20 und deren Verbindung mit dem Skrotum-Bügel. Indirekt wird aber durch die dortige Anordnung der Spannösen beim Spannen der Rundgummischnur der Ringquerschnitt an der Penisbasis elastisch stark eingeengt und dadurch der Blutrückfluß im Penis behindert. Durch diese Strangulierung wird im Penis 2 im Verein mit der Wirkung aus dem Gefühlskontakt an der großen freien Oberfläche eine erektionsartige Füllung des völlig impotenten Penis 2 erreicht, desgleichen eine Anschwellung der Eichel 7 durch die dortige Strangulierung in der Spannschlaufe, die den Penis 2 gegen die Längsstäbe 18, 19, 20 zieht. Diese erektionsartige Schwellung und innere Spannung erlaubt überhaupt erst den Orgasmus des eigentlich impotenten Penis 2 und damit die Ejakulation. Die gelegentliche Ejakulation aber erscheint für den Erhalt der Gesundheit der Prostata im Alter dringend erforderlich.

Damit ergibt sich für die Gestaltung einer praktischen, wirksamen und sicheren Außenprothese keine andere Möglichkeit als die beschriebene und gewählte Form und Mechanik.

### Bezugszeichenliste

- 1: Korb
- 2: Penis
- 3: Bügel
- 4: Skrotum
- 5: Ring
- 6: Auflage
- 7: Eichel
- 8: Langloch
- 9: Rundgummischnur
- 10: Schlaufe
- 11: Moosgummischnur
- 12: Aufhängung
- 13: Aufhängung
- 14: Brücke
- 15: Oval
- 16: Vorhaut
- 17: Vollgummischeibe
- 18: Längsstab
- 19: Längsstab
- 20: Längsstab

## Patentansprüche

1. Außenprothese zur Stützung des Penis (2) bei teilweiser oder völliger Impotenz, bestehend aus einem den Penis (2) umfassenden, steif ausgebildeten, in der Vagina gleitfähigen, als Korb (1) ausgebildeten Hohlkörper, der auf seiner bei Benutzung dem Körper zugewandten Seite zum Durchführen des Skrotums (4) von oben her einen in Horizontalebene offenen Bügel (3) hat und nahe seines vorderen Endes eine Auflage (6) für den Penis (2) aufweist, **dadurch gekennzeichnet**, daß die Auflage (6) mit einer von oben her über den Hals der Eichel (7) des Penis (2) zu ziehende, diesen auf der Auflage (6) fixierende und einschnürende Schlaufe (10) versehen und die Schlaufe (10) Teil einer geschlossenen Rundgummischnur (9) ist, welche zum Spannen der Schlaufe (10) in eine Aufhängung (12, 13) eines rückwärtigen Ringes (5) einhängbar ist.

2. Außenprothese nach Anspruch 1, **dadurch gekennzeichnet**, daß der Bügel (3) fest und unbeweglich ausgebildet ist und ihm sich zum Durchführen des Penis (2) der in Vertikalebene offene rückwärtige Ring (5) anschließt

3. Außenprothese nach Anspruch 2, **dadurch gekennzeichnet**, daß die Aufhängungen (12, 13) an zwei Seiten des Ringes (5) vorgesehen und zur Erzeugung eines zweiten, den Penis (2) an seiner Basis durch die Rundgummischnur (9) einschnürenden Bereiches ausgebildet sind.

4. Außenprothese nach zumindest einem der vorangehenden Ansprüche, **dadurch gekennzeichnet**, daß die Auflage (6) zur Durchführung der Schlaufe (10) ein in Querrichtung verlaufendes Langloch (8) hat.

5. Außenprothese nach zumindest einem der vorangehenden Ansprüche, **dadurch gekennzeichnet**, daß die Schlaufe (10) aus querelastischer Moosgummischnur (11) besteht.

6. Außenprothese nach Anspruch 4, **dadurch gekennzeichnet**, daß der Querschnitt der Schlaufe (10) größer ist als der des Langloches (8) und der Rundgummischnur (9).

7. Außenprothese nach Anspruch 6, **dadurch gekennzeichnet**, daß auf der Rundgummischnur (9) zu beiden Seiten der Schlaufe (10) jeweils eine gegen den jeweiligen Beginn der Schlaufe anliegende Vollgummischeibe (17) sitzt.

8. Außenprothese nach zumindest einem der vorangehenden Ansprüche, **dadurch gekennzeichnet**, daß der Korb (1) zusätzlich zu dem Ring (5) eine Brücke (14) in einem mittleren Bereich hat.

9. Außenprothese nach zumindest einem der vorangehenden Ansprüche, **dadurch gekennzeichnet**, daß der Korb (1) drei Längsstäbe (18, 19, 20) hat, welche vom Ring (5) nach vorn führen, wobei die beiden oberen Längsstäbe (18, 19) im mittleren Bereich durch die Brücke (14) miteinander verbunden sind und im vorderen Bereich mit einem schräg nach unten gerichteten Oval (15) eine Einheit bilden und daß dieses Oval (15) die Auflage (6) für die Eichel (7) bildet.

10. Außenprothese nach Anspruch 9, **dadurch gekennzeichnet**, daß das Oval (15) mindestens doppelt so lang ist, wie das für den fixierten Eichelhals notwendig ist.

11. Außenprothese nach zumindest einem der vorangehenden Ansprüche, **dadurch gekennzeichnet**, daß der Korb (1) bananenförmig nach vorn oben gekrümmt verläuft.

## Claims

1. External prosthesis for supporting the penis (2) in partial or complete impotence, consisting of a hollow body which surrounds the penis (2), is rigid in design, is capable of sliding in the vagina, is designed as a basket (1), has a hoop (3) which is open in a horizontal plane for passage of the scrotum (4) from above on its side facing the body during use and has, close to its front end, a support (6) for the penis (2), characterised in that the support (6) is provided with a loop (10) which is to be pulled over the neck of the glans (7) of the penis (2) from above, and is to fix and tie it on the support (6) and the loop (10) is part of a closed round rubber cord (9) which can be suspended in a suspension means (12, 13) of a rear ring (5) for tensioning the loop (10).

2. External prosthesis according to claim 1, characterised in that the loop (3) is rigid and unmovable in design and the rear ring (5) which is open in a vertical plane adjoins it for the passage of the penis (2).

3. External prosthesis according to claim 2, characterised in that the suspension means (12, 13) are provided on two sides of the ring (5) and are designed to produce a second region which ties the penis (2) at its base by means of the round rubber cord (9).

4. External prosthesis according to at least one of the preceding claims, characterised in that the support (6) has a transversely extending slot (8) for the passage of the loop (10).

5. External prosthesis according to at least one of the preceding claims, characterised in that the loop (10) consists of transverse elastic rubber cord (11) with a moss-like covering.

6. External prosthesis according to claim 4, characterised in that the cross section of the loop (10) is greater than that of the slot (8) and of the round rubber cord (9).

7. External prosthesis according to claim 6, characterised in that a solid rubber disc (17) lying against the respective beginning of the loop in each case rests on the round rubber cord (9) on either side of the loop (10).

8. External prosthesis according to at least one of the preceding claims, characterised in that the basket (1) has a bridge (14) in a central region in addition to the ring (5).

9. External prosthesis according to at least one of the preceding claims, characterised in that the basket (1) has three longitudinal rods (18, 19, 20) which lead forwardly from the ring (5), wherein the two upper longitudinal rods (18, 19) are connected to one another in the central region by the bridge (14) and, in the front region, form a unit with an obliquely downwardly directed oval (15) and in that this oval (15) forms the support (6) for the glans (7).

10. External prosthesis according to claim 9, characterised in that the oval (15) is at least twice as long as necessary for the fixed glans neck.

11. External prosthesis according to at least one of the preceding claims, characterised in that the basket (1) is curved upwardly at the front in the form of a banana.

## Revendications

1. Prothèse extérieure destinée à renforcer le pénis (2) dans le cas d'impuissance partielle ou totale, constituée d'une pièce creuse conformée en panier, de constitution rigide, qui entoure le pénis (2) et peut glisser à l'intérieur du vagin, cette pièce creuse possédant, sur son côté orienté vers le corps lors de son utilisation, un étrier (3) ouvert dans le plan horizontal pour permettre le passage du scrotum (4), et présentant, a proximité de son extrémité avant, un appui (6) pour le pénis, caractérisée en ce que l'appui (6) est muni d'une boucle (10) devant être passée, à partir du haut, sur le col du gland (7) du pénis (2), fixant ce dernier sur l'appui (6) et le ceinturant, ladite boucle (10) étant une partie d'une cordelette (9) circulaire fermée en caoutchouc, laquelle peut être accrochée dans une suspension (12, 13) d'une bague arrière (5) en vue de tendre la boucle (10).

2. Prothèse extérieure selon la revendication 1, caractérisée en ce que l'étrier (3) est fixe et immobile et fait suite à la bague (5) ouverte dans le plan vertical pour permettre le passage du pénis (2).

3. Prothèse extérieure selon la revendication 2, caractérisée en ce que les suspensions (12, 13) sont prévues sur deux côtés de la bague (5) et réalisées de manière à produire une deuxième zone ceinturant le pénis (2) à sa base sous l'effet de la cordelette (9).

4. Prothèse extérieure selon au moins l'une des revendications précédentes, caractérisée en ce que l'appui (6) possède un trou oblong (8) s'étendant en direction transversale pour permettre le passage de la boucle (10).

5. Prothèse extérieure selon au moins l'une des revendications précédentes, caractérisée en ce que la boucle (10) est constituée par une cordelette (11) en caoutchouc mousse, élastique dans le sens transversal.

6. Prothèse extérieure selon la revendication 4, caractérisée en ce que la section transversale de la boucle (10) est plus grande que celle du trou oblong (8) et de la cordelette circulaire (9) en caoutchouc.

7. Prothèse extérieure selon la revendication 6, caractérisée en ce que sur la cordelette circulaire (9) en caoutchouc, est montée, des deux côtés respectivement de la boucle (10), une rondelle (17) en caoutchouc plein qui vient s'appliquer contre la zone respective de ladite boucle.

8. Prothèse extérieure selon au moins l'une des revendications précédentes, caractérisée en ce que le panier (11) possède, en plus de la bague (5), un pont 14 situé dans une zone médiane.

9. Prothèse extérieure selon au moins l'une des revendications précédentes, caractérisée en ce que le panier (11) possède trois barres longitudinales (18, 19, 20) qui sont dirigées vers l'avant à partir de la bague (5), les deux barres supérieures (18, 19) étant reliées mutuellement, dans la zone médiane, par le pont (14) et forment une seule unité, dans la zone antérieure, avec un ovale (15) orienté vers le bas, et en ce que cet ovale (15) constitue l'appui (6) pour le gland (7).

10. Prothèse extérieure selon la revendication 9, caractérisée en ce que l'ovale (15) présente une longueur égale au moins au double de celle nécessaire pour le col du gland fixé en place.

11. Prothèse extérieure selon au moins l'une des revendications précédentes, caractérisée en ce que le panier (11) s'étend en forme de banane recourbée en haut vers l'avant.
